(19) Europäisches Patentamt
European Patent Office
Office européen des brevets

(11) Numéro de publication : **0 352 182 B1**

(12) # FASCICULE DE BREVET EUROPEEN

(45) Date de publication du fascicule du brevet : **05.05.93 Bulletin 93/18**

(21) Numéro de dépôt : **89402040.3**

(22) Date de dépôt : **18.07.89**

(51) Int. Cl.$^5$ : **C07D 487/22,** C25B 3/02, G01N 24/10, // (C07D487/22, 259:00, 209:00, 209:00, 209:00, 209:00)

(54) **Phtalocyanines de lithium octasubstituées, leur procédé de préparation et leur utilisation en magnétométrie rpe.**

(30) Priorité : **20.07.88 FR 8809831**

(43) Date de publication de la demande : **24.01.90 Bulletin 90/04**

(45) Mention de la délivrance du brevet : **05.05.93 Bulletin 93/18**

(84) Etats contractants désignés : **DE FR GB**

(56) Documents cités :
**EP-A- 0 188 941**
**CHEMICAL ABSTRACTS, vol. 95, 1981, page 737, abrégé n 179991k, Columbus, Ohio, US; E.A. CUELLAR et al.: "Synthesis and characterization of metallo and metal-free octaalkylphthalocyanines and uranyl decaalkylsuperphthalocyaninates"**

(56) Documents cités :
**JOURNAL OF THE CHEMICAL SOCIETY, CHEM. COMMUN., 1983, pages 962-963, Londres, GB; H. SUGIMOTO et al.: "Synthesis and molecular structure of a lithium complex of the phthalocyanine radical"**

(73) Titulaire : **COMMISSARIAT A L'ENERGIE ATOMIQUE**
**31/33, rue de la Fédération**
**F-75015 Paris (FR)**

(72) Inventeur : **Moussavi, Mehdi**
**20, rue Joseph Rolland**
**F-38120 Saint Egreve (FR)**
Inventeur : **Secourgeon, Liliane**
**3 rue de l'Ancienne Ferme**
**F-38120 Saint Egreve (FR)**

(74) Mandataire : **Mongrédien, André et al**
**c/o BREVATOME 25, rue de Ponthieu**
**F-75008 Paris (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne de nouvelles phtalocyanines de lithium octasubstituées, en particulier sous la forme de composés radicalaires stables, utilisables pour la mesure de champs magnétiques par résonance paramagnétique électronique (RPE).

Depuis quelques années, on a effectué de nombreuses recherches pour trouver des radicaux organiques, métalliques ou semiconducteurs, utilisables en magnétométrie (RPE), et on a développé en particulier des systèmes ioniques tels que les sels de fluoranthène et les composés de tétrathiafulvalène- tétracyanoquinodiméthane, comme il est décrit par E.Dormann et al dans Journal of Magnetism and Magnetic Materials, 54-57, (1986), p. 1315-1316.

L'utilisation de ces composés en magnétométrie présente certains inconvénients. En effet, les sels d'ions radicaux appartenant à la famille du fluoranthène ou (TTF-CNQ) sont des systèmes à plusieurs composants et les problèmes liés à leurs stoechiométries imprévisibles et à leur manque de stabilité chimique et thermique sont inévitables. De plus, les propriétés de conduction électrique de ces matériaux perturbent les mesures de champ magnétique par effet de peau.

Aussi, des recherches ont été entreprises pour trouver d'autres matériaux plus performants.

Parmi les matériaux susceptibles de présenter des propriétés appropriées pour une utilisation en magnétométrie, on a envisagé l'utilisation des phtalocyanines radicalaires telles que la phtalocyanine de lithium, comme il est décrit par Turek et al dans Solid State Communications, Vol. 63, n° 8, p. 741-744, 1987. Cette phtalocyanine de lithium radicalaire peut être préparée par oxydation électrochimique, par voie potentiostatique, de la phtalocyanine dilithiée, comme il est décrit par Sujimoto et al dans J. Chem. Soc. Chem. Commun., 1986, p. 962-963.

La phtalocyanine de lithium radicalaire paraît intéressante pour réaliser des mesures magnétiques, mais le signal obtenu avec ce composé est insuffisant pour justifier son application industrielle. Par ailleurs, la phtalocyanine de lithium radicalaire est très sensible à l'action de l'oxygène comme l'ont décrit Turek et al dans le document précité, ce qui conduit à un élargissement de la raie RPE.

De plus, la préparation de phtalocyanine de lithium radicalaire par oxydation électrochimique potentiostatique ne permet pas d'obtenir des cristaux ayant des propriétés satisfaisantes. En effet, la génèse des cristaux de phtalocyanine de lithium démarre à une valeur d'intensité maximum pour diminuer ensuite. Ceci entraîne une croissance rapide et non reproductible de phtalocyanine de lithium radicalaire sous forme de poudre ou de très petits cristaux, qui comportent des défauts dans le meilleur des cas.

La présente invention a précisément pour objet de nouvelles phtalocyanines de lithium, utilisables en magnétométrie RPE, qui pallient les inconvénients de la phtalocyanine de lithium radicalaire décrite ci-dessus.

Selon l'invention, la phtalocyanine de lithium est une phtalocyanine de lithium octasubstituée répondant à la formule :

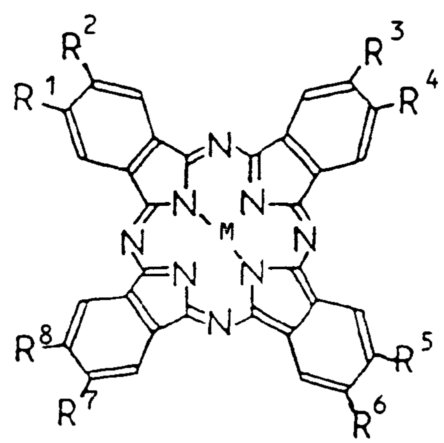

(I)

dans laquelle $R^1$, $R^2$, $R^3$, $R^4$, $R^5$, $R^6$, $R^7$ et $R^8$ qui sont identiques ou différents, représentent un radical alkyle ou alcoxy ayant de 1 à 3 atomes de carbone, non substitué ou substitué par un ou plusieurs atomes de fluor, et M représente Li ou $Li_2$.

Lorsque M représente $Li_2$, il s'agit du précurseur dilithié des phtalocyanines de lithium octasubstituées radicalaire de l'invention.

Lorsque M représente Li , il s'agit de la phtalocyanine de lithium radicalaire PcLi octasubstituées utilisable en magnétométrie RPE.

La présence sur cette phtalocyanine de lithium radicalaire des substituants $R^1$ à $R^8$ permet d'améliorer les propriétés de la phtalocyanine pour son utilisation en magnétométrie RPE et de faciliter sa préparation à partir du précurseur dilithié.

En effet, le signal RPE de la phtalocyanine de lithium radicalaire non substituée est élargi par la présence d'oxygène comme on l'a vu précédemment. Aussi, ce composé n'est utilisable en magnétométrie que sous vide d'air. Cependant, quel que soit le niveau de vide du conditionnement d'un échantillon de phtalocyanine de lithium radicalaire, il reste de l'oxygène résiduel dans des sites d'inclusion existants dans les cristaux de PcLi radicalaire.

Selon l'invention, on évite cet inconvénient en substituant en périphérie la phtalocyanine par des groupements de taille et de nature adéquates qui permettent de combler l'espace entre les molécules de manière à rendre plus compacte la structure cristalline et empêcher la pénétration de l'oxygène. Les substituants sont des groupements donneurs d'électrons qui ont par ailleurs une influence favorable sur la réaction d'oxydation du précurseur dilithié dans le procédé de préparation de la phtalocyanine de lithium radicalaire octasubstituée par oxydation électrochimique du précurseur dilithié.

Les substituants permettant d'obtenir cet effet sont des groupements donneurs d'électrons qui ne doivent pas avoir un effet donneur important qui se traduirait par une dégradation de la stabilité de la phtalocyanine de lithium radicalaire. Il ne faut pas non plus que l'effet donneur d'électrons soit insuffisant car ceci ne se traduirait pas par une amélioration sensible des propriétés de la phtalocyanine de lithium radicalaire. L'effet donneur peut être contrôlé par la substitution d'un ou plusieurs atomes d'hydrogène par le nombre correspondant d'atomes de fluor sur 2 à 8 atomes de carbone de la phtalocyanine octaméthoxy ou plus dans le cas où sur la phtalocyanine considérée, le groupement substituant comporte plusieurs atomes de carbone.

Selon l'invention, les substituants sont des radicaux alkyle ou alcoxy ayant de 1 à 3 atomes de carbone. Dans ces radicaux, un ou plusieurs atomes d'hydrogène peuvent être remplacés par un ou plusieurs atomes de fluor afin d'ajuster l'effet donneur global de ces substituants sur la phtalocyanine.

Généralement, $R^1$ à $R^8$ représentent des radicaux identiques, ayant de préférence un seul atome de carbone, comme les radicaux méthoxy, méthyle et trifluorométhoxy.

De préférence, on utilise le radical méthoxy car il s'agit d'un donneur mésomère d'électrons qui active le macrocycle phtalocyanine et facilite la perte d'un électron par le précurseur dilithié.

En effet, on a constaté qu'en utilisant comme substituant un groupement attracteur comme le fluor, on désactive fortement le macrocycle en rendant l'oxydation du précurseur dilithié très difficile.

Les précurseurs dilithiés des phtalocyanines de lithium radicalaire de l'invention peuvent être préparés par des procédés classiques à partir d'un dérivé disubstitué, ou d'un ou plusieurs dérivés disubstitués du benzène répondant aux formules :

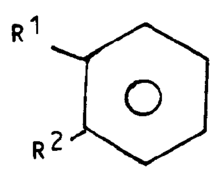
(II)

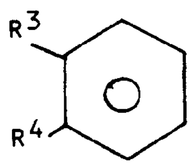
(III)

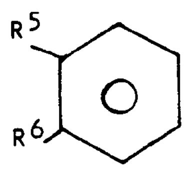
(IV)

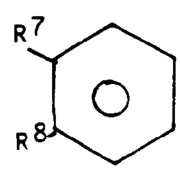
(V)

dans lesquelles $R^1$, $R^2$, $R^3$, $R^4$, $R^5$, $R^6$, $R^7$ et $R^8$ ont la signification donnée ci-dessus.

Pour cette préparation, on transforme ces dérivés disubstitués en dérivés tétrasubstitués répondant aux formules :

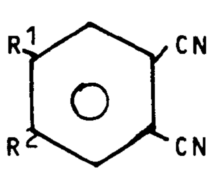
(VI)

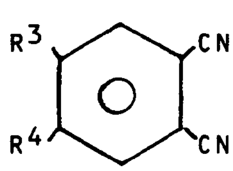
(VII)

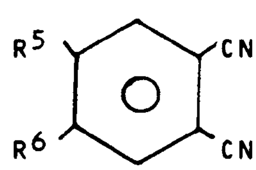
(VIII)

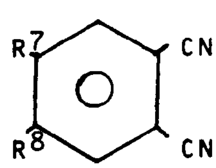
(IX)

puis on fait réagir un ou plusieurs de ces dérivés tétrasubstitués avec du lithium pour former la $PcLi_2$ octasubstituée correspondante.

Dans le cas de chacun des dérivés disubstitués de formule II à V décrits ci-dessus, le dérivé tétrasubstitué est obtenu en effectuant les réactions suivantes :

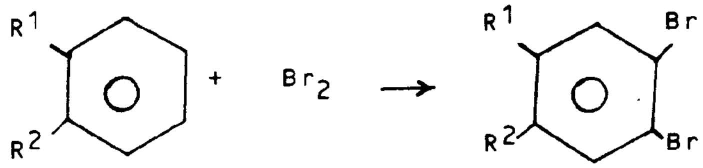
(1)

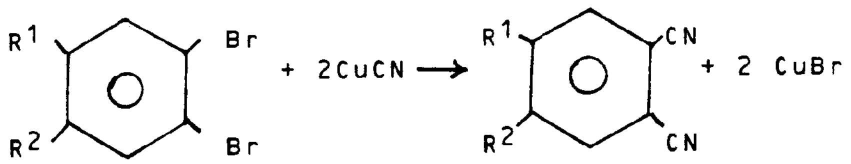
(2)

La première réaction (1) qui consiste à préparer le dérivé dibromé peut être effectuée par action du brome en présence de catalyseurs tels que de la poudre de fer et de l'iode, comme il est décrit par E. Klingsberg dans Synthesis (1972), p. 29-30, dans le cas de la préparation du dérivé dibromo de l'orthoxylène.

La réaction (2) peut être effectuée en faisant réagir le dérivé obtenu par la réaction 1 avec du cyanure de cuivre en léger excès et en solution diluée, en utilisant un solvant comme le diméthylformamide et en opérant au reflux.

La préparation de la phtalocyanine dilithiée octasubstituée peut être effectuée ensuite en faisant réagir un ou plusieurs dérivés répondant aux formules VI à IX avec du lithium, en solution dans un alcool tel que l'alcool amylique. Lorsqu'on effectue cette réaction avec 2, 3 ou 4 dérivés différents mentionnés ci-dessus, on obtient un mélange de phtalocyanines dilithiées octasubstituées qu'il est nécessaire de séparer ensuite pour isoler les molécules ayant des formules identiques. Cette séparation peut être effectuée par chromatographie liquide sur colonne d'alumine ou de silice.

Généralement, on part d'un seul dérivé disubstitué du benzène dans lequel $R^1$ et $R^2$ peuvent être identiques ou différents. Dans ce cas, on obtient exclusivement la phtalocyanine octasubstituée dilithiée de formule :

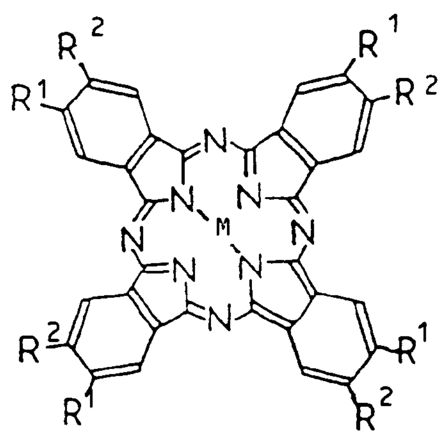
(X)

dans laquelle $R^1$ et $R^2$ ont la signification donnée ci-dessus et M représente $Li_2$.

Selon l'invention, on passe du dérivé dilithié octasubstitué à la phtalocyanine de lithium radicalaire octasubstituée par oxydation électrochimique réalisée dans des conditions différentes de celles utilisées antérieurement pour passer de la phtalocyanine dilithiée à la phtalocyanine de lithium radicalaire.

Aussi, l'invention a aussi pour objet un procédé de préparation de phtalocyanine de lithium radicalaire octasubstituée qui consiste à oxyder une phtalocyanine dilithiée octasubstituée de formule :

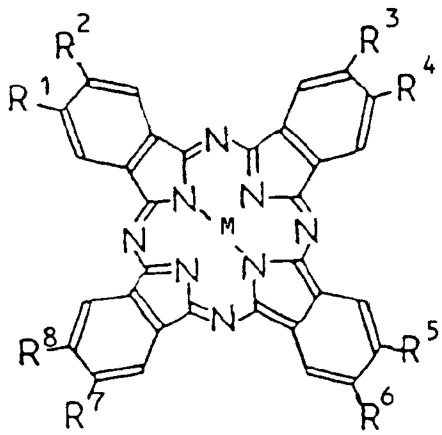

(I)

dans laquelle $R^1$, $R^2$, $R^3$, $R^4$, $R^5$, $R^6$, $R^7$ et $R^8$ ont la signification donnée ci-dessus et M représente $Li_2$, par oxydation monoélectronique par voie galvanostatique.

Dans ce procédé, on applique un courant faible au début de la réaction d'oxydation de manière à ralentir la formation des germes ; on contrôle ainsi mieux la vitesse de cristallogénèse et par conséquent la qualité cristalline du produit obtenu. La réaction est ainsi plus reproductible et permet d'obtenir des cristaux plus performants.

En effet, la substitution périphérique de la phtalocyanine conduit à un déplacement dans le sens cathodique des potentiels d'oxydo-réduction, c'est-à-dire que les composés octasubstitués par des radicaux alkyle ou alcoxy s'oxydent plus facilement que la phtalocyanine non substituée. Toutefois, il est bon de limiter le nombre de carbones des groupes substituants à 3 pour ne pas rendre incontrôlable la dernière étape de synthèse :

$$R_8PcLi_2 \rightarrow R_8P\dot{c}Li + 1e + 1Li^+$$

Lorsqu'on réalisait, selon l'art antérieur, l'oxydation électrochimique du dérivé dilithié selon la méthode potentiostatique, on imposait aux bornes des électrodes plongées dans une solution de phtalocyanine dilithiée un potentiel correspondant à celui du palier de la courbe de variations de l'intensité du courant i en fonction du potentiel. De la sorte, la génèse des cristaux de phtalocyanine de lithium radicalaire démarrait à une valeur d'intensité maximum pour diminuer ensuite. On obtenait ainsi une croissance rapide et non reproductible de phtalocyanine de lithium radicalaire sous forme de poudre ou de très petits cristaux comportant des défauts.

L'invention sera mieux comprise à la lecture de la description qui suit donnée bien entendu à titre illustratif et non limitatif en référence au dessin annexé qui représente une cellule électrochimique à trois compartiments pour mettre en oeuvre le procédé de l'invention.

Sur cette figure (1), on voit que la cellule électrochimique comprend un premier compartiment cathodique (1) associé à une cathode (3), un deuxième compartiment anodique (5) associé à une anode (7) et un compartiment intermédiaire (9) situé entre le compartiment cathodique et le compartiment anodique. Le compartiment cathodique est délimité par une enveloppe en verre (11) munie à sa partie inférieure d'une pastille poreuse en verre fritté (13).

Le compartiment anodique (5) est fermé à sa partie inférieure par une pastille poreuse (15) en verre fritté ; et le compartiment intermédiaire (9) est délimité dans la cellule entre les pastilles poreuses en verre fritté (13 et 15). Les compartiments anodiques et cathodiques peuvent être obturés à leur partie supérieure par des bouchons étanches (17 et 19).

Les électrodes (3 et 7) peuvent être raccordées à un générateur de courant continu.

Pour réaliser l'oxydation électrochimique du dérivé dilithié octasubstitué de phtalocyanine, on introduit celui-ci en solution dans un solvant comportant un électrolyte support dans le compartiment anodique (5) de la cellule. On remplit le compartiment cathodique avec le même solvant et le même électrolyte support, puis on dégaze soigneusement les deux compartiments et on ferme hermétiquement la cellule. On applique ensuite une différence de potentiel entre les électrodes (3 et 7) de façon à faire passer des courants faibles au début de l'oxydation et l'on augmente ensuite les courants à des moments déterminés pour recueillir les cristaux présentant la meilleure qualité.

Le solvant utilisé peut être une cétone telle que la butanone-2, l'électrolyte support peut être un sel d'ammonium quaternaire tel que le perchlorate de tétrabutylammonium.

Les exemples suivants de préparation d'octaméthoxyphtalocyanine de lithium radicalaire et d'octaméthylphtalocyanine de lithium radicalaire sont donnés bien entendu à titre non limitatif pour illustrer l'invention.

**Exemple 1** : Préparation de l'octaméthoxy phtalocyanine de lithium radicalaire.

a) Préparation du 1,2-dibromo-4,5-diméthoxybenzène à partir du 1,2-diméthoxybenzène.

Cette réaction correspond au schéma suivant :

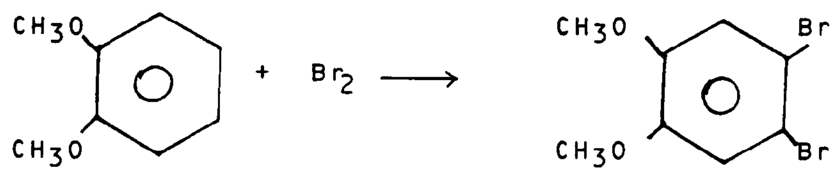

On ajoute 156g (0,98mol) de brome sur une période de cinq heures à 68g (0,49 mol) de 1,2-diméthoxybenzène à une température de 5 à 10°C en utilisant de la poudre de fer et de l'iode comme catalyseurs à raison de 1g de fer et 1 cristal d'iode.

Vers la fin de l'addition de brome, on facilite l'agitation du mélange en diluant avec du dichlorométhane que l'on élimine ensuite partiellement par distillation sur un bain de vapeur. On filtre ensuite le produit obtenu et on le sèche. On lave le produit brut avec du méthanol froid (4°C) jusqu'à élimination de la couleur rouge brun caractéristique du brome. On obtient ainsi 106g de 1,2-dibromo-4,5-diméthoxybenzène de couleur beige clair, ce qui correspond à un rendement de 73%.

b) Préparation du 1,2-dicyano-4,5 méthoxy-benzène.

Cette réaction correspond au schéma réactionnel suivant :

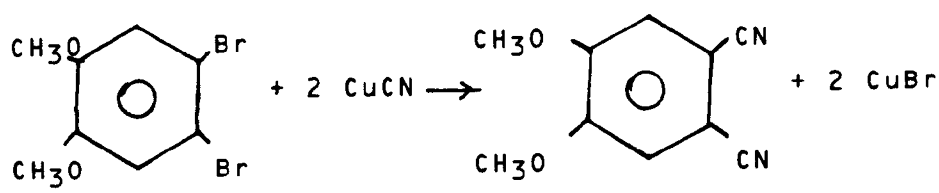

On porte au reflux pendant cinq heures un mélange de 106g (0,36mol) de 1,2-dibromo-4,5-diméthoxybenzène, 98g (1,06mol) de cyanure de cuivre et 300ml de diméthylformamide, puis on refroidit le mélange et on le verse dans 500ml d'ammoniaque à 30%. On mélange encore pendant 10 minutes, puis on filtre et on lave le résidu avec de l'eau et on le sèche. On extrait le produit par de l'éther pendant 24 heures en utilisant un appareil de Soxhlet, et on évapore le solvant. On obtient ainsi 40g de 1,2-dicyano-4,5 diméthoxybenzène sous forme de paillettes blanches, ce qui correspond à un rendement de 58%.

c) Préparation d'octaméthoxyphtalocyanine dilithiée.

On ajoute 38g (0,2mol) de 1,2-dicyano-4,5 diméthoxybenzène à une solution de 2g de lithium dans 150cm$^3$ d'alcool amylique. On chauffe ensuite le mélange, une réaction fortement exothermique prend place et de l'octaméthoxyphtalocyanine de dilithium se sépare. On fait bouillir le mélange pendant 90 minutes, puis on le refroidit et on le dilue avec 0,5 litre de benzène, puis on le maintient pendant trois heures à 4°C. On filtre alors le mélange et on obtient ainsi 8g d'octaméthoxyphtalocyanine de dilithium que l'on extrait avec de l'acétone qui a été séché au préalable sur du sulfate de sodium. Par évaporation sous pression réduite, on obtient 5g d'octaméthoxyphtalocyanine de dilithium sous la forme d'un dépôt cristallin, ce qui correspond à un rendement de 13%.

d) Préparation de l'octaméthoxyphtalocyanine de lithium radicalaire.

Pour cette préparation, on utilise la cellule électrochimique représentée sur la figure 1 pour réaliser l'oxydation monoélectronique de l'octaméthoxyphtalocyanine de dilithium obtenu précédemment, par voie galvanostatique. On nettoie tout d'abord soigneusement et on sèche à l'étuve à 60°C la cellule électrochimique et les électrodes (3) et (7) qui sont constituées toutes deux par des fils de platine ayant un diamètre de 1mm et

une longueur de 3,5cm. On introduit alors dans le compartiment anodique (5) une solution de 90mg de $(CH_3O)_8PcLi_2$ et de 500mg de perchlorate de tétrabutylammonium (TBAP) dans 100ml de butanone-2. On remplit alors le compartiment cathodique (1)avec 50ml de butanone-2 contenant 500mg de TBAP. On procède ensuite au dégazage par bullage d'argon pur dans les deux compartiments, puis on ferme hermétiquement la cellule à l'aide des bouchons (17) et (19). On applique alors les courants suivants aux bornes de la cellule :

0,5μA pendand 15 heures,
2,5μA pendant 24 heures,
5μA pendant 72 heures, et
10μA pendant 24 heures.

On obtient ainsi des particules polycristallines d'octaméthoxyphtalocyanine de lithium radicalaire formées à l'anode. On récupère ces particules polycristallines, puis on recueille les cristaux qui restent sur l'anode par décantation après trois lavages successifs avec des portions de 100ml d'acétone pur et sec. On obtient ainsi 20mg au total d'octaméthoxyphtalocyanine de lithium radicalaire, ce qui correspond à un rendement de 22,5%.

**Exemple 2** : Préparation d'octaméthyl phtalocyanine de lithium radicalaire.

On suite le même mode opératoire que dans l'exemple 1 pour préparer cette phtalocyanine sauf que l'on part du 1,2-diméthylbenzène, soit du O-xylène.

On obtient ainsi l'octaméthyl phtalocyanine de lithium radicalaire avec un rendement similaire à celui obtenu dans l'exemple 1.

L'octaméthoxyphtalocyanine de lithium radicalaire et l'octaméthyl phtalocyanine de lithium radicalaire des exemples 1 et 2 présentent de bonnes propriétés pour une utilisation en magnétométrie, en particulier dans un magnétomètre tel que celui décrit dans le brevet français FR-A- 2 603 384.

Dans le tableau joint, on a regroupé les résultats obtenus avec divers matériaux en ce qui concerne la mesure de la pente maximale du signal RPE à 1,8MHz, c'est-à-dire la pente de la partie centrale de la courbe dérivée du spectre d'absorption du matériau en utilisant un spectromètre RPE à bas champ. Les valeurs obtenues permettent des comparaisons quantitatives des matériaux pour leur utilisation en magnétométrie. On constate au vu de ce tableau que les phtalocyanines de lithium radicalaires octasubstituées de l'invention présentent des performances supérieures à celles des matériaux connus. De plus, le signal RPE de $(CH_3O)_8PcLi$ ou de $(CH_3)_8PcLi$ a une pente maximum sur une large plage de champs d'excitation et de détection, ce qui implique une très nette facilité de réglage pour l'obtention des performances maximum lors de l'utilisation en magnétométrie de ces matériaux.

En effet, la pente maximum pour le signal de tous les matériaux à RPE fine est observée pour des valeurs précises de puissance, d'excitation et de détection.

Dans ce tableau, on a également indiqué la stabilité des matériaux à 20°C, et on remarque que les phtalocyanines de lithium radicalaires octasubstituées de l'invention présentent également une bonne stabilité.

**TABLEAU**

| Matériau | Pente maximale du signal RPE à 1,8 MHz par mg | Stabilité (à 20°C) |
|---|---|---|
| 2,2-diphényl-1-picrylhydrazyle | $0.1.10^{-9}$ | très stable |
| Tétracyano quinodiméthane diquinoléinium | $45.10^{-9}$ | très stable |
| Hexafluorophosphate de fluoranthène | $70.10^{-9}$ | instable |
| Hexafluorophosphate de naphtalène | $90.10^{-9}$ | très instable |
| PcLi | $40.10^{-9}$ | très stable |
| $(CH_3O)_8PcLi$ | $100.10^{-9}$ | très stable |
| $(CH_3)_8PcLi$ | $90.10^{-9}$ | très stable |

## Revendications

1. Phtalocyanine de lithium octasubstituée répondant à la formule :

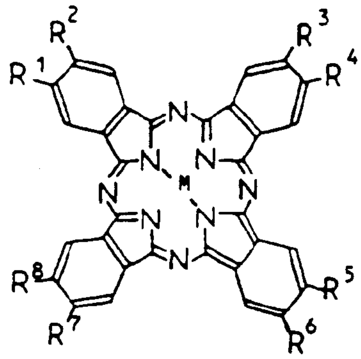

(I)

dans laquelle $R^1$, $R^2$, $R^3$, $R^4$, $R^5$, $R^6$, $R^7$ et $R^8$ qui sont identiques ou différents, représentent un radical alkyle ou alcoxy ayant de 1 à 3 atomes de carbone, non substitué ou substitué par un ou plusieurs atomes de fluor, et M représente Li ou $Li_2$.

2. Phtalocyanine de lithium selon la revendication 1, caractérisé en ce que $R^1$ à $R^8$ sont identiques.

3. Phtalocyanine de lithium selon la revendication 2, caractérisé en ce que $R^1$, $R^2$, $R^3$, $R^4$, $R^5$, $R^6$, $R^7$ et $R^8$ représentent le radical méthoxy.

4. Phtalocyanine de lithium selon la revendication 2, caractérisé en ce que $R^1$, $R^2$, $R^3$, $R^4$, $R^5$, $R^6$, $R^7$ et $R^8$ représentent le radical méthyle.

5. Phtalocyanine de lithium octasubstituée répondant à la formule :

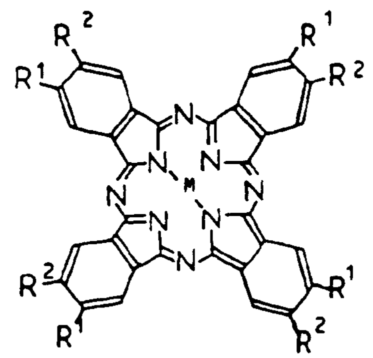

(x)

dans laquelle $R^1$ et $R^2$ qui peuvent être identiques ou différents, représentent un radical alkyle ou alcoxy de 1 à 3 atomes de carbone, et M représente Li ou $Li_2$.

6. Phtalocyanine de lithium octasubstituée selon la revendication 5, caractérisé en ce que $R^1$ et $R^2$ qui sont identiques ou différents, représentent le radical méthyle ou méthoxy.

7. Phtalocyanine de lithium octasubstituée selon l'une quelconque des revendications 1 à 6, caractérisée en ce que M est Li.

8. Procédé de préparation d'une phtalocyanine de lithium octasubstituée de formule :

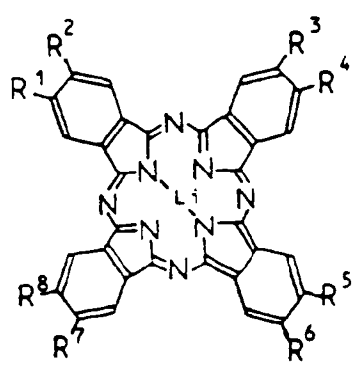

dans laquelle $R^1$, $R^2$, $R^3$, $R^4$, $R^5$, $R^6$, $R^7$, et $R^8$ qui peuvent être identiques ou différents représentent un radical alkyle ou alcoxy de 1 à 3 atomes de carbone, caractérisé en ce qu'il consiste à oxyder monoélectroniquement par voie galvanostatique la phtalocyanine octasubstituée de dilithium répondant à la formule :

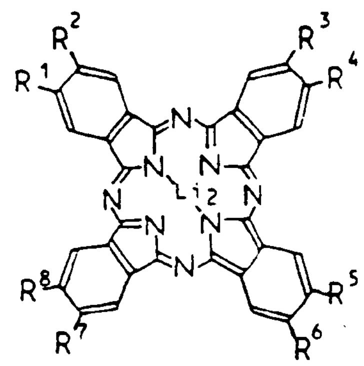

dans laquelle $R^1$ à $R^8$ ont la signification donnée ci-dessus.

**9.** Procédé selon la revendication 8, caractérisé en ce que $R^1$ à $R^8$ représentent le radical méthoxy.

**10.** Procédé selon la revendication 8, caractérisé en ce que $R^1$ et $R^8$ représentent le radical méthyle.

**11.** Utilisation en magnétométrie par résonance paramagnétique électronique d'une phtalocyanine de lithium octasubstituée radicalaire selon la revendication 7.

## Patentansprüche

**1.** Octasubstituiertes Lithiumphthalocyanin entsprechend der Formel:

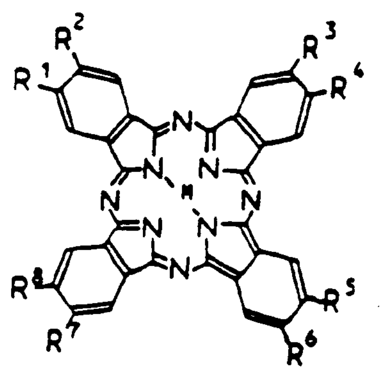

(I)

in welcher $R^1$, $R^2$, $R^3$, $R^4$, $R^5$, $R^6$, $R^7$ und $R^8$, die gleich oder verschieden sind, einen Alkylrest oder Alkoxyrest mit 1 bis 3 Kohlenstoffatomen, der nicht substituiert oder durch ein oder mehrere Fluoratome substituiert ist, bedeuten, und M Li oder $Li_2$ bedeutet.

**2.** Lithiumphthalocyanin nach Anspruch 1, **dadurch gekennzeichnet**, daß $R^1$ bis $R^8$ gleich sind.

**3.** Lithiumphthalocyanin nach Anspruch 2, **dadurch gekennzeichnet**, daß $R^1$, $R^2$, $R^3$, $R^4$, $R^5$, $R^6$, $R^7$ und $R^8$ den Methoxyrest bedeuten.

**4.** Lithiumphthalocyanin nach Anspruch 2, **dadurch gekennzeichnet**, daß $R^1$, $R^2$, $R^3$, $R^4$, $R^5$, $R^6$, $R^7$ und $R^8$ den Methylrest bedeuten.

**5.** Octasubstituiertes Lithiumphthalocyanin entsprechend der Formel:

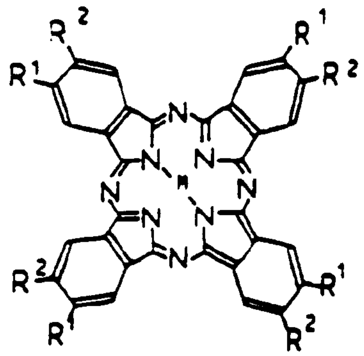

(x)

in welcher $R^1$ und $R^2$, die gleich oder verschieden sein können, einen Alkylrest oder Alkoxyrest mit 1 bis 3 Kohlenstoffatomen bedeuten und M Li oder $Li_2$ bedeutet.

6. Octasubstituiertes Lithiumphthalocyanin nach Anspruch 5, **dadurch gekennzeichnet**, daß $R^1$ und $R^2$, die gleich oder verschieden sind, den Methyl- oder Methoxyrest bedeuten.

7. Octasubstituiertes Lithiumphthalocyanin nach einem der Ansprüche 1 bis 6, **dadurch gekennzeichnet**, daß M Li ist.

8. Verfahren zur Herstellung eines octasubstituierten Lithiumphthalocyanins der Formel:

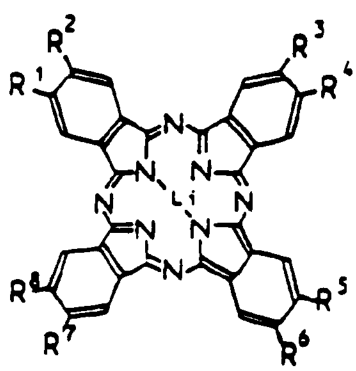

in welcher $R^1$, $R^2$, $R^3$, $R^4$, $R^5$, $R^6$, $R^7$ und $R^8$, die gleich oder verschieden sein können, einen Alkylrest oder Alkoxyrest mit 1 bis 3 Kohlenstoffatomen bedeuten, **dadurch gekennzeichnet**, daß es das monoelektronische Oxidieren auf galvanostatischem Weg des octasubstituierten Dilithiumphthalocyanins entsprechend der Formel:

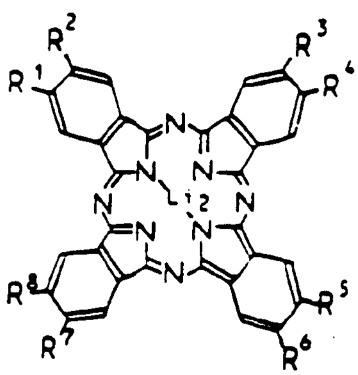

in welcher $R^1$ bis $R^8$ die oben angegebene Bedeutung haben, beinhaltet.

9. Verfahren nach Anspruch 8, **dadurch gekennzeichnet**, daß $R^1$ bis $R^8$ den Methoxyrest bedeuten.

10. Verfahren nach Anspruch 8, **dadurch gekennzeichnet**, daß $R^1$ bis $R^8$ den Methylrest bedeuten.

11. Verwendung eines radikalartigen octasubstituierten Lithiumphthalocyanins nach Anspruch 7 in der Elektronenspinresonanz-Spektroskopie.

## Claims

1. Octasubstituted lithium phthalocyanine responding to the formula :

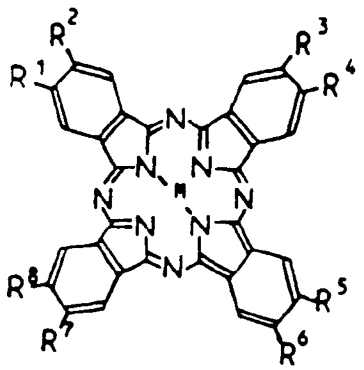

(I)

in which $R^1$, $R^2$, $R^3$, $R^4$, $R^5$, $R^6$, $R^7$ and $R^8$, which are identical or different, represent an alcoxy or alkyl radical having from 1 to 3 carbon atoms, possibly substituted by one or more fluorine atoms, and M represents Li or $Li_2$.

2. Lithium phthalocyanine according to claim 1, wherein $R^1$ to $R^8$ are identical.

3. Lithium phthalocyanine according to claim 2, wherein $R^1$, $R^2$, $R^3$, $R^4$, $R^5$, $R^6$, $R^7$ and $R^8$ represent the methoxy radical.

4. Lithium phthalocyanine according to claim 2, wherein $R^1$, $R^2$, $R^3$, $R^4$, $R^5$, $R^6$, $R^7$ and $R^8$ represent the methyl radical.

5. Octasubstituted lithium phthalocyanine responding to the formula :

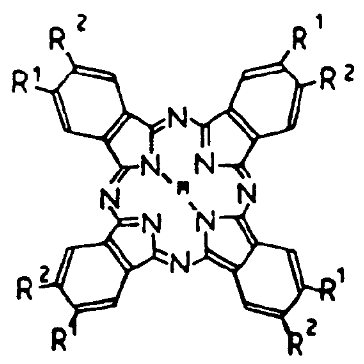

(x)

in which $R^1$ and $R^2$ , which may be identical or different, represent an alcoxy or alkyl radical with from 1 to 3 carbon atoms, and M represents Li or $Li_2$.

6. Octasubstituted lithium phthalocyanine according to claim 5, wherein $R^1$ and $R^2$ , which are identical or different, represent the methoxy or methyl radical.

7. Octasubstituted lithium phthalocyanine according to any one of claims 1 to 6, wherein M is Li.

8. Method for preparing an octasubstituted lithium phthalocyanine with the formula :

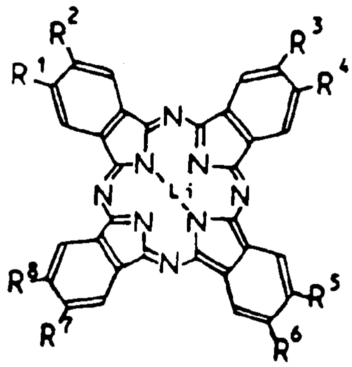

in which $R^1$, $R^2$, $R^3$, $R^4$, $R^5$, $R^6$, $R^7$ and $R^8$ , which may be identical or different, represent an alcoxy or alkyl radical with from 1 to 3 carbon atoms, wherein said method consists of mono of monoelectronically by galvanostatic means the octasubstituted dilithium phthalocyanine responding to the formula :

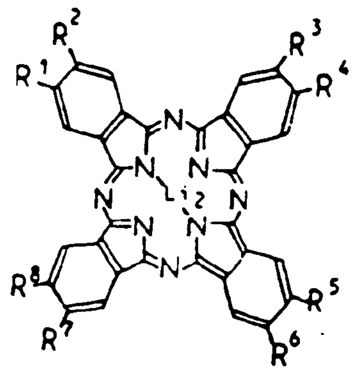

in which R and R have the significance given above.

**9.** Method according to claim 8, wherein R to R represent the methoxy radical.

**10.** Method according to claim 8, wherein $R^1$ to $R^8$ represent the methyl radical.

**11.** Use in electronic paramagnetic resonannce magnetometry of a radical-like octasubstituted lithium phthalocyanine according to claim 7.

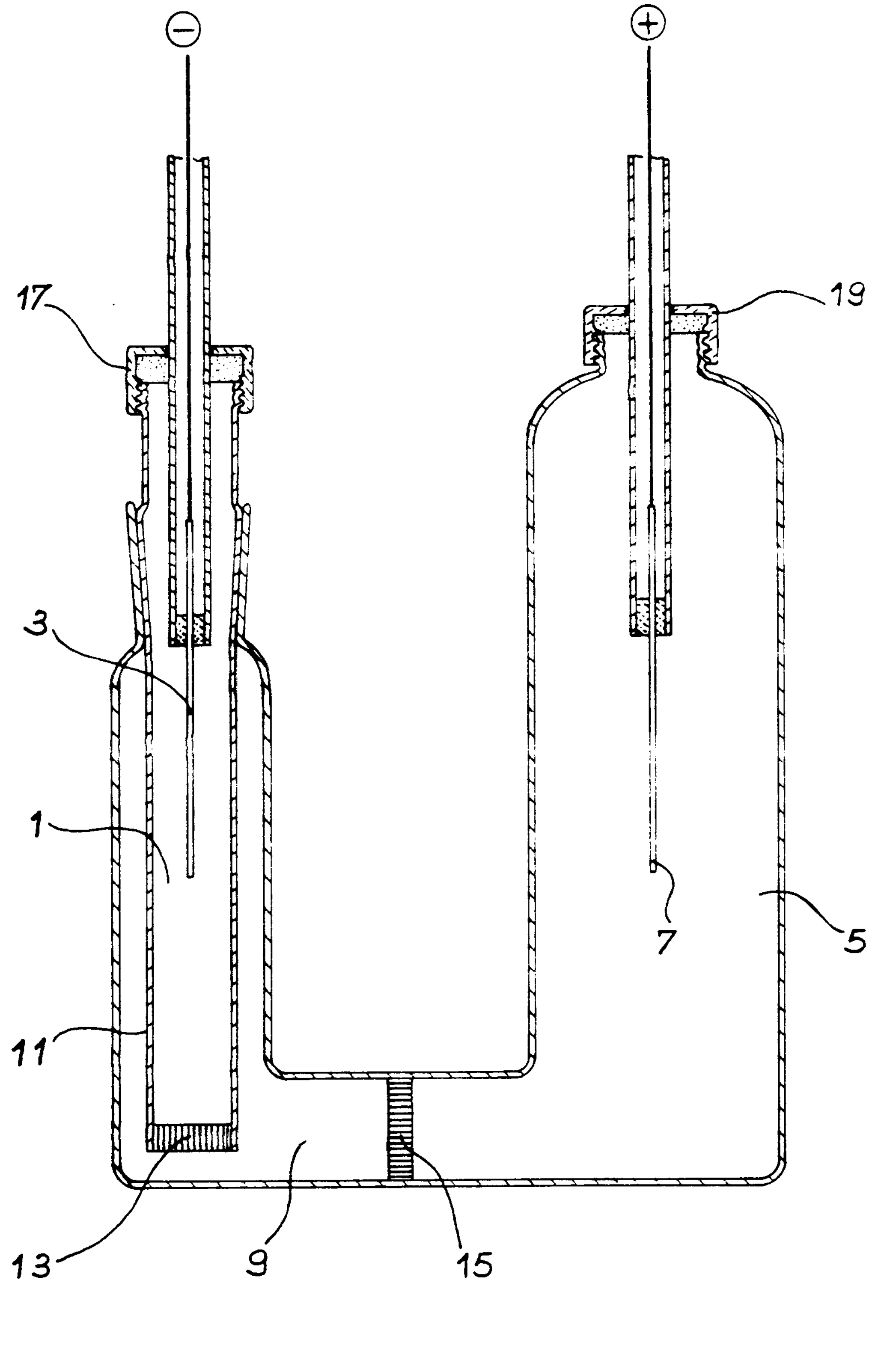
⊖
⊕
17
19
3
1
7
5
11
13
9
15